# EUROPEAN PATENT APPLICATION

(11) **EP 4 115 793 A1**
(43) Date of publication of application: **11.01.2023**
(21) Application number: 21763809.7
(22) Date of filing: 26.01.2021
(51) Int. Cl.: A61B 1/045, A61B 1/00, A61B 1/06, G02B 23/24

(54) **ENDOSCOPE SYSTEM, CONTROL METHOD, AND CONTROL PROGRAM**

(30) Priority: 06.03.2020 JP 2020038996
(71) Applicant: FUJIFILM Corporation, Tokyo 106-8620 (JP)
(72) Inventor: SHIMOMURA Koji, Ashigarakami-gun, Kanagawa 258-8538 (JP)
(74) Representative: Hughes, Andrea Michelle
(86) International application number: PCT/JP2021/002669
(87) International publication number: WO 2021/176890

(57) **Abstract**

Provided are an endoscope system, a control method, and a control program that can perform imaging with special light while suppressing the deterioration of the quality of the motion picture display. The light source device 5 repeats an operation of continuously emitting illumination light having a first characteristic in a first period over a plurality of consecutive imaging operation frames, and then emitting illumination light having a second characteristic in a second period over at least one imaging operation frame. The display 7 displays a motion picture using the captured image obtained from an imaging element 23, displays the motion picture based on the captured image of the first period for the first period, and displays the motion picture based on the captured image of a period different from the second period for the second period.

## Description

### BACKGROUND OF THE INVENTION

### 1. Field of the Invention

The present invention relates to an endoscope system, a control method, and a control program.

### 2. Description of the Related Art

In the related art, an endoscope system is known in which continuous imaging is performed while irradiating a subject with normal light, such as white light, to display a live image. In addition, there is known an endoscope system that performs continuous imaging while irradiating a subject with special light, such as narrow band light, and performs analysis such as image-enhanced endoscopy (IEE).

JP2016-019569A discloses an endoscope system that acquires a special light image generated by performing imaging while a subject is irradiated with special light, and a normal light image generated by performing imaging while the subject is irradiated with normal light. JP2011-234844A discloses an endoscope apparatus that generates a special light image based on an acquired white light image by using a spectroscopic estimation technology and simultaneously acquires the white light image and the special light image.

### SUMMARY OF THE INVENTION

However, with the related art, it is not possible to perform imaging with special light while suppressing a deterioration of a quality of motion picture display.

For example, in a case in which imaging is performed while switching between the normal light and the special light, a frame rate of the captured image in a case of the normal light used for the motion picture display is decreased, so that the frame rate of the motion picture display is decreased. In addition, in a case in which the captured image during the irradiation with the special light is used for the motion picture display, a color is changed in the motion picture display, which causes a sense of discomfort to a user.

The present invention has been made in view of the circumstances described above, and is to provide an endoscope system, a control method, and a control program that can perform imaging with special light while suppressing the deterioration of the quality of the motion picture display.

An aspect of the present invention relates to an endoscope system comprising a light source, an imaging element, a display, and a processor, in which the light source is able to switch between a plurality of types of illumination light having different characteristics to perform irradiation, and the processor is configured to repeat an operation of continuously emitting illumination light having a first characteristic from the light source in a first period over a plurality of consecutive imaging operation frames, and then emitting illumination light having a second characteristic different from the first characteristic from the light source in a second period over at least one imaging operation frame, and acquire a captured image obtained from the imaging element, and cause the display to display, for the first period, the motion picture based on the captured image of the first period and to display, for the second period, the motion picture based on the captured image of a period different from the second period.

In addition, another aspect of the present invention relates to a control method of an endoscope system including a light source, an imaging element, a display, and a processor, in which the light source is able to switch between a plurality of types of illumination light having different characteristics to perform irradiation, the method comprising repeating an operation of causing the light source to continuously emit illumination light having a first characteristic in a first period over a plurality of consecutive imaging operation frames, and then emit illumination light having a second characteristic different from the first characteristic in a second period over at least one imaging operation frame, and acquiring a captured image obtained from the imaging element, and causing the display to display, for the first period, the motion picture based on the captured image of the first period and to display, for the second period, the motion picture based on the captured image of a period different from the second period.

In addition, still another aspect of the present invention relates to a control program that controls an endoscope system including a light source, an imaging element, a display, and a processor, in which the light source is able to switch between a plurality of types of illumination light having different characteristics to perform irradiation, the program causing a computer to execute a process comprising repeating an operation of causing the light source to continuously emit illumination light having a first characteristic in a first period over a plurality of consecutive imaging operation frames, and then emit illumination light having a second characteristic different from the first characteristic in a second period over at least one imaging operation frame, and acquiring a captured image obtained from the imaging element, and causing the display to display, for the first period, the motion picture based on the captured image of the first period and to display, for the second period, the motion picture based on the captured image of a period different from the second period.

According to the present invention, it is possible to provide the endoscope system, the control method, and the control program that can perform imaging with the special light while suppressing the deterioration of the quality of the motion picture display.

### BRIEF DESCRIPTION OF THE DRAWINGS

Fig. 1 is a view showing an example of an endoscope apparatus 100 which is one embodiment of the present invention.
Fig. 2 is a schematic view showing an internal configuration of the endoscope apparatus 100 shown in Fig. 1.
Fig. 3 is a diagram showing an example of a spectrum of light generated by a light source device 5 shown in Fig. 2.
Fig. 4 is a schematic plan view showing a schematic configuration of an imaging element 23 shown in Fig. 2.
Fig. 5 is a diagram showing an example of functional blocks of a system control unit 44 of a signal processing unit 42 shown in Fig. 2.
Fig. 6 is a diagram showing an example of a screen displayed on a display 7.
Fig. 7 is a diagram showing an example of switching of illumination light in the endoscope apparatus 100.
Fig. 8 is a diagram (part 1) showing an example of a change in an image due to switching of the illumination light.
Fig. 9 is a diagram (part 2) showing an example of the change in the image due to switching of the illumination light.
Fig. 10 is a diagram showing an example of interpolation of a display frame during irradiation with special light.
Fig. 11 is a diagram showing another example of the interpolation of the display frame during the irradiation with the special light.

### DESCRIPTION OF THE PREFERRED EMBODIMENTS

In the following, an embodiment of the present invention will be described with reference to the drawings.

### <Endoscope Apparatus 100 Which Is One Embodiment of Present Invention>

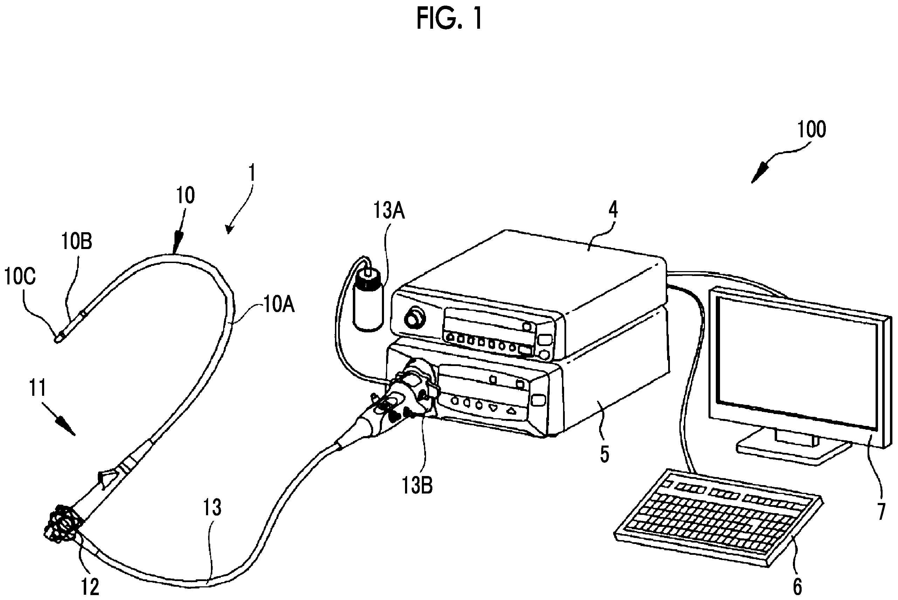

Fig. 1 is a view showing an example of an endoscope apparatus 100 which is one embodiment of the present invention.

The endoscope apparatus 100 is an example of an endoscope system according to the embodiment of the present invention. As shown in Fig. 1, the endoscope apparatus 100 comprises an endoscope 1, and a control device 4 and a light source device 5 to which the endoscope 1 is connected. The light source device 5 is an example of a light source capable of switching between a plurality of types of illumination light having different characteristics to perform irradiation.

A display 7 that displays a captured image or the like obtained by imaging an inside of a subject by the endoscope 1 and an input unit 6, which is an interface for inputting various types of information to the control device 4 are connected to the control device 4. The control device 4 controls the endoscope 1, the light source device 5, and the display 7.

The display 7 has a display surface on which display pixels are two-dimensionally arranged, and pixel data constituting image data is drawn on each display pixel on the display surface, thereby performing display of an image based on the image data. The display 7 constitutes a display unit that switches the display image in accordance with the command from the control device 4.

The endoscope 1 includes an insertion part 10 which is a tubular member extending in one direction and is inserted into the subject, an operating part 11 which is provided in a base end part of the insertion part 10 and includes an operation member for performing an observation mode switching operation, an imaging recording operation, a forcep operation, an air supply/water supply operation, and a suction operation, an angle knob 12 provided adjacent to the operating part 11, and a universal cord 13 including connector portions 13A and 13B that detachably connect the endoscope 1 to the control device 4 and the light source device 5, respectively.

It should be noted that, although not shown in Fig. 1, various channels, such as a forcep hole for inserting forceps for sampling a living body tissue, such as cells or polyps, an air supply/water supply channel, and a suction channel, are provided inside the operating part 11 and the insertion part 10.

The insertion part 10 is composed of a flexible part 10A having flexibility, a bendable part 10B provided at a distal end of the flexible part 10A, and a hard distal end part 10C provided at a distal end of the bendable part 10B.

The bendable part 10B is configured to be bendable by a rotational movement operation of the angle knob 12. Depending on the site of the subject in which the endoscope 1 is used, the bendable part 10B can be bent in any direction and at any angle, and the distal end part 10C can be directed in a desired direction.

### <Internal Configuration of Endoscope Apparatus 100 Shown in Fig. 1>

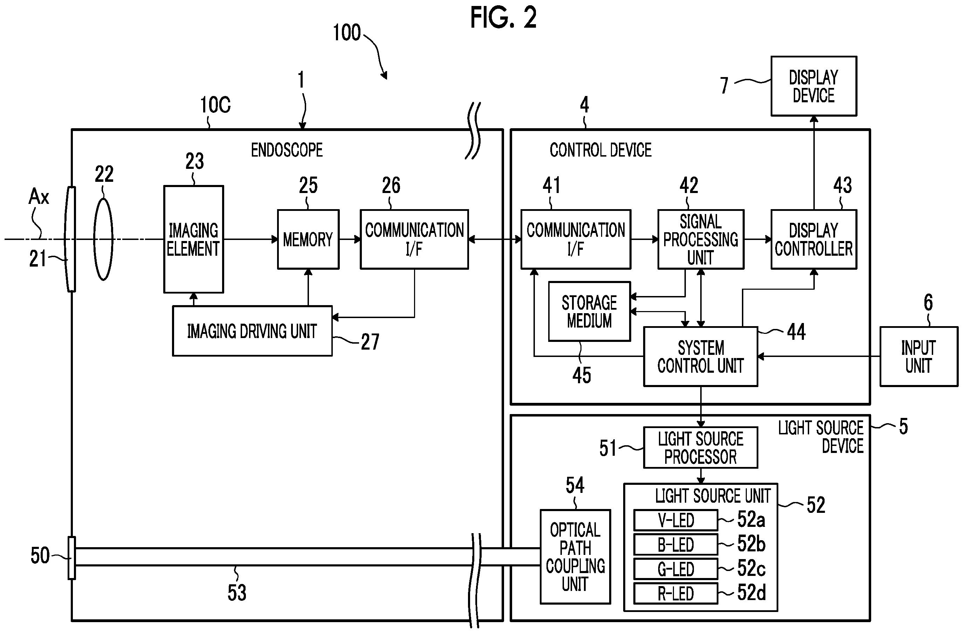

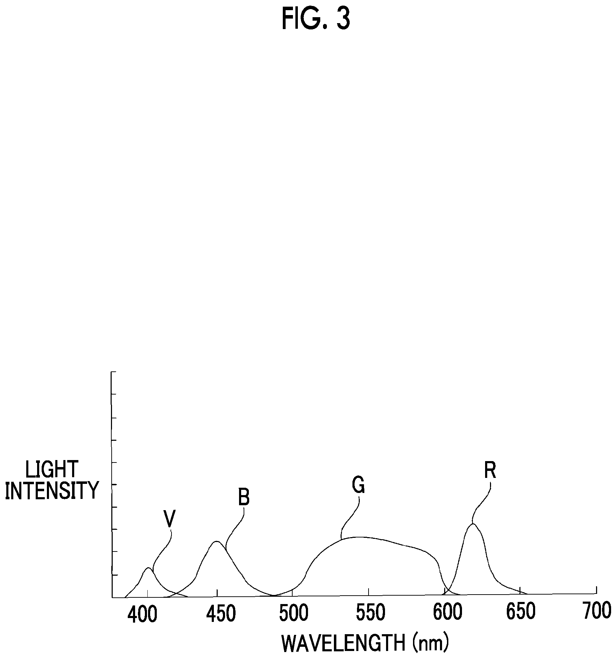

Fig. 2 is a schematic view showing an internal configuration of the endoscope apparatus 100 shown in Fig. 1. Fig. 3 is a diagram showing an example of a spectrum of light generated by the light source device 5 shown in Fig. 2.

The light source device 5 can switch between normal light and special light as illumination light and perform irradiation. The normal light is light having an emission spectrum suitable for recognition by a human, such as a doctor, such as white light. The special light is light having an emission spectrum suitable for image analysis by a computer, such as IEE, which has a different emission spectrum from the normal light.

Specifically, the light source device 5 comprises a light source processor 51, a light source unit 52, and an optical path coupling unit 54. The light source processor 51 is connected to the system control unit 44 of the control device 4, and controls the light source unit 52 based on the command from the system control unit 44.

The light source unit 52 has, for example, a plurality of semiconductor light sources, each of which is turned on or off, and in a case in which the light source unit 52 is turned on, the emission amount of each semiconductor light source is controlled to emit the illumination light for illuminating an observation target. In the present embodiment, the light source unit 52 has LEDs of four colors, a violet light emitting diode (V-LED) 52a, a blue light emitting diode (B-LED) 52b, a green light emitting diode (G-LED) 52c, and a red light emitting diode (R-LED) 52d.

By independently controlling each of the V-LED 52a, the B-LED 52b, the G-LED 52c, and the R-LED 52d, the light source processor 51 can emit violet light V, blue light B, green light G, or red light R by independently changing a light amount. As shown in Fig. 3, the V-LED 52a generates the violet light V of which a central wavelength is in a range of 405±10 nm and a wavelength range is in a range of 380 to 420 nm. The B-LED 52b generates the blue light B of which a central wavelength is in a range of 450±10 nm and a wavelength range is in a range of 420 to 500 nm. The G-LED 52c generates the green light G of which a wavelength range is in a range of 480 to 600 nm. The R-LED 52d generates the red light R of which a central wavelength is in a range of 620 to 630 nm and a wavelength range is in a range of 600 to 650 nm.

In addition, in a case of irradiation with the normal light, the light source processor 51 controls each of the LEDs 52a to 52d to emit the white light in which a light amount ratio of the violet light V, the blue light B, the green light G, and the red light R is Vc:Bc:Gc:Rc. It should be noted that Vc, Bc, Gc, Rc > 0.

In addition, in a case of irradiation with the special light, the light source processor 51 controls each of the LEDs 52a to 52d to emit the special light in which the light amount ratio of the violet light V, the blue light B, the green light G, and the red light R as short-wavelength narrow-band light is Vs:Bs:Gs:Rs.

The light amount ratio Vs:Bs:Gs:Rs is different from the light amount ratio Vc:Bc:Gc:Rc used in a case of the irradiation with the normal light, and is appropriately determined in accordance with the observation purpose. For example, in a case in which superficial blood vessels are enhanced, it is preferable to make Vs larger than Bs, Gs, and Rs, and in a case in which mesopelagic blood vessels are enhanced, it is preferable to make Gs larger than Vs, Gs, and Rs.

The optical path coupling unit 54 combines each light emitted from the V-LED 52a, the B-LED 52b, the G-LED 52c, and the R-LED 52d, and emits the combined light as the illumination light. The illumination light emitted from the optical path coupling unit 54 of the light source unit 52 enters a light guide 53 to be described below built in the universal cord 13, and is emitted to the subject through an illumination lens 50 provided at the distal end part 10C of the insertion part 10.

In the distal end part 10C of the endoscope 1, an imaging optical system including an objective lens 21 and a lens group 22, an imaging element 23 that images the subject through the imaging optical system, a memory 25, such as a random access memory (RAM), a communication interface (I/F) 26, an imaging driving unit 27, and the light guide 53 for guiding the illumination light emitted from the light source unit 52 to the illumination lens 50 are provided.

The light guide 53 extends from the distal end part 10C to the connector portion 13A of the universal cord 13. The illumination light emitted from the light source unit 52 of the light source device 5 can enter the light guide 53 in a state in which the connector portion 13A of the universal cord 13 is connected to the light source device 5.

A charge coupled device (CCD) image sensor or a complementary metal oxide semiconductor (CMOS) image sensor is used as the imaging element 23. In the present embodiment, the imaging element 23 is the CMOS using a rolling shutter.

The imaging element 23 has a light-receiving surface on which a plurality of pixels are two-dimensionally arranged, and converts an optical image formed on the light-receiving surface by the imaging optical system described above into an electrical signal (imaging signal) in each pixel. Moreover, the imaging element 23 converts the converted imaging signal from an analog signal into a digital signal having a predetermined number of bits, and outputs the imaging signal converted into the digital signal to the memory 25. For example, an imaging element on which a color filter, such as an elementary color or a complementary color, is mounted, is used as the imaging element 23. A set of the imaging signals output from the pixels of the light-receiving surface of the imaging element 23 is referred to as a captured image signal.

The imaging element 23 may be disposed at the distal end part 10C in a state in which the light-receiving surface is perpendicular to an optical axis Ax of the objective lens 21, or may be disposed at the distal end part 10C in a state in which the light-receiving surface is parallel to the optical axis Ax of the objective lens 21.

The imaging optical system provided in the endoscope 1 is composed of optical members (including the lens group 22 described above), such as a lens and a prism, which are present on an optical path of the light from the subject between the imaging element 23 and the objective lens 21, and the objective lens 21. There is also a case in which the imaging optical system is composed of only the objective lens 21.

The memory 25 transitorily records the digital imaging signal output from the imaging element 23.

The communication I/F 26 is connected to a communication interface (I/F) 41 of the control device 4. The communication I/F 26 transmits the imaging signal recorded in the memory 25 to the control device 4 through a signal line in the universal cord 13.

The imaging driving unit 27 is connected to the system control unit 44 of the control device 4 via the communication I/F 26. The imaging driving unit 27 drives the imaging element 23 and the memory 25 based on the command from the system control unit 44 received by the communication I/F 26.

The control device 4 comprises the communication I/F 41, which is connected to the communication I/F 26 of the endoscope 1 by the universal cord 13, a signal processing unit 42, a display controller 43, the system control unit 44, and a recording medium 45.

The communication I/F 41 receives the imaging signal transmitted from the communication I/F 26 of the endoscope 1 to transmit the imaging signal to the signal processing unit 42.

The signal processing unit 42 has a memory that transitorily records the imaging signal received from the communication I/F 41 built therein, and performs processing (image processing, such as demosaicing processing or gamma correction processing) on the captured image signal that is a set of the imaging signals recorded in the memory to generate captured image information in such a format that recognition processing to be described below or the like can be performed. The captured image information generated by the signal processing unit 42 is recorded on the recording medium 45, such as a hard disk or a flash memory.

The display controller 43 displays a captured image based on the captured image information generated by the signal processing unit 42 on the display 7. A coordinate of each pixel data constituting the captured image information generated by the signal processing unit 42 is managed in association with a coordinate of any of the display pixels constituting the display surface of the display 7.

The system control unit 44 controls each unit of the control device 4, and transmits the command to the imaging driving unit 27 of the endoscope 1 and the light source processor 51 of the light source device 5, and integrally controls the entire endoscope apparatus 100. For example, the system control unit 44 performs the control of the imaging element 23 via the imaging driving unit 27. In addition, the system control unit 44 performs the control of the light source unit 52 via the light source processor 51.

The system control unit 44 or the signal processing unit 42 includes various processors that execute a program to perform processing, a RAM, and a read only memory (ROM).

Examples of various processors include a central processing unit (CPU), which is a general-purpose processor that executes the program to perform various types of processing, a programmable logic device (PLD), which is a processor of which the circuit configuration can be changed after the manufacture, such as a field programmable gate array (FPGA), and a dedicated electric circuit, which is a processor having the circuit configuration specially designed for executing specific processing, such as an application specific integrated circuit (ASIC).

More specifically, the structure of these various processors is an electric circuit in which circuit elements, such as semiconductor elements, are combined.

The system control unit 44 or the signal processing unit 42 may be composed of one of the various processors, or may be composed of a combination (for example, a combination of a plurality of FPGAs or a combination of a CPU and an FPGA) of two or more processors of the same type or different types.

### <Schematic Configuration of Imaging Element 23 Shown in Fig. 2>

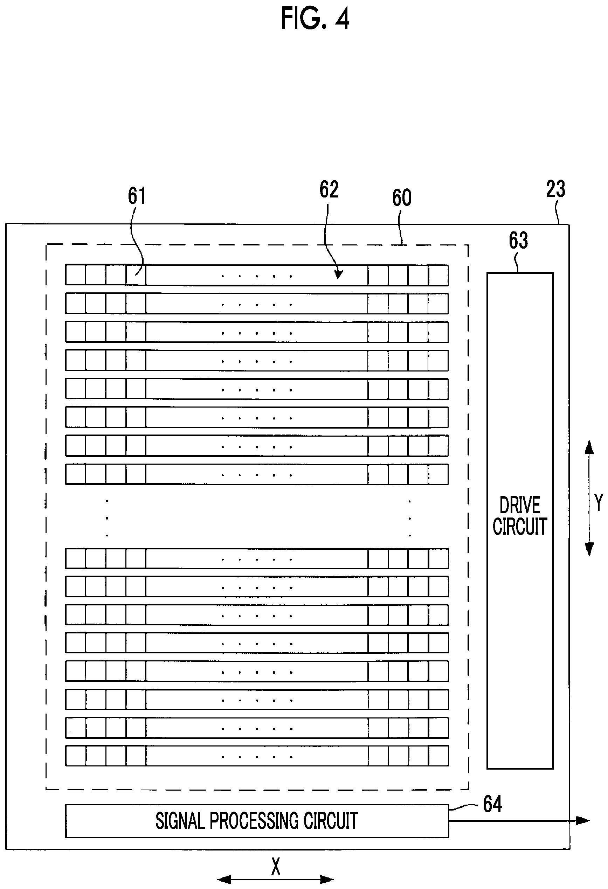

Fig. 4 is a schematic plan view showing a schematic configuration of the imaging element 23 shown in Fig. 2.

The imaging element 23 comprises an imaging surface 60 on which a plurality of pixel rows 62 consisting of a plurality of pixels 61 arranged in a row direction X are arranged in a column direction Y orthogonal to the row direction X, a drive circuit 63 that drives the pixels 61 arranged on the imaging surface 60, and a signal processing circuit 64 that processes the pixel signal read out from each pixel 61 of the pixel row 62 arranged on the imaging surface 60 into the signal line. The imaging surface 60 constitutes the light-receiving surface.

In the following, in Fig. 4, an end portion of the imaging surface 60 on one end side (upper side in Fig. 4) in the column direction Y is referred to as an upper end, and an end portion of the imaging surface 60 on the other end side (lower side in Fig. 4) in the column direction Y is referred to as a lower end.

The drive circuit 63 shown in Fig. 4 independently drives each pixel row 62 based on the signal from the imaging driving unit 27, and performs the reset of each pixel 61 included in the pixel row 62 (discharge of charge accumulated in the photoelectric conversion element), and the reading out of the pixel signal in accordance with the charge accumulated in the photoelectric conversion element of each pixel 61 into the signal line.

The signal processing circuit 64 shown in Fig. 4 performs sampling two correlation pile processing on the pixel signal read out from each pixel 61 of the pixel row 62 into the signal line, converts the pixel signal subjected to the sampling two correlation pile processing into the digital signal, and outputs the converted pixel signal. The signal processing circuit 64 is controlled by the imaging driving unit 27.

The signal processing unit 42 performs the signal processing, such as the demosaicing processing and the gamma correction processing, on the pixel signal output from the imaging element 23 to generate the captured image information.

The endoscope apparatus 100 is equipped with a continuous imaging mode that continuously generates a plurality of pieces of the captured image information in accordance with one imaging instruction. In the continuous imaging mode, the system control unit 44 drives the imaging element 23 by the imaging driving unit 27 by a rolling shutter system to image the subject.

The driving of the rolling shutter system includes the rolling reset driving and the rolling read-out driving. The rolling reset driving is driving in which processing of resetting each pixel 61 of the pixel row 62 and starting the exposure of each pixel 61 is sequentially performed while changing the pixel row 62. The rolling read-out driving is driving in which processing of reading out the signal from each pixel 61 of the exposed pixel row 62 and terminating the exposure of the pixel row 62 is sequentially performed while changing the pixel row 62.

### <Functional Blocks of System Control Unit 44 of Signal Processing Unit 42 Shown in Fig. 2>

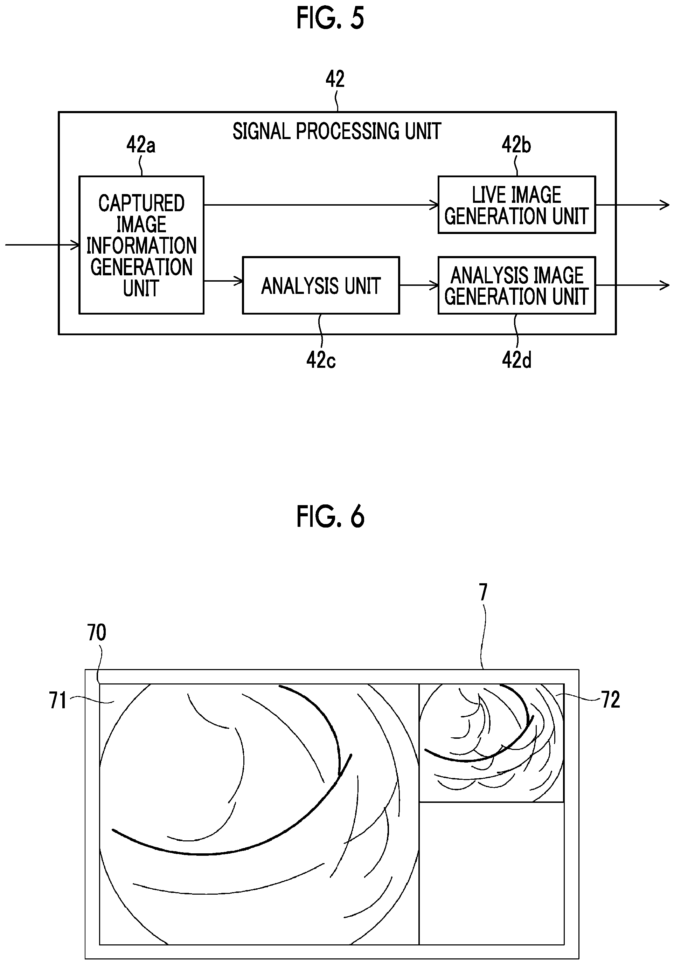

Fig. 5 is a diagram showing an example of functional blocks of the system control unit 44 of the signal processing unit 42 shown in Fig. 2.

A processor of the signal processing unit 42, for example, executes a control program stored in the ROM built in the signal processing unit 42 to comprise a captured image information generation unit 42a, a live image generation unit 42b, an analysis unit 42c, and an analysis image generation unit 42d.

The captured image information generation unit 42a generates the captured image information by performing the image processing, such as the demosaicing processing or the gamma correction processing, on the imaging signal obtained by imaging of the imaging element 23. The captured image information generation unit 42a outputs the captured image information based on the imaging signal obtained by imaging during the irradiation with the normal light to the live image generation unit 42b as an imaging frame in the generated captured image information, and outputs the captured image information based on the imaging signal obtained by imaging during the irradiation with the special light to the analysis unit 42c as an imaging frame. The imaging frame is the imaging signal obtained by one imaging.

The live image generation unit 42b generates live image information for displaying the live image based on the imaging frame output from the captured image information generation unit 42a, and outputs the generated live image information to the display controller 43 (see Fig. 2) as the captured image information. The live image is the motion picture that displays a result of continuous imaging by the imaging element 23 in real time.

The analysis unit 42c performs the analysis (image analysis) based on the imaging frame output from the captured image information generation unit 42a, and outputs a result of the analysis to the analysis image generation unit 42d. As an example, the analysis unit 42c performs contour extraction of the captured image as the analysis. For example, the analysis unit 42c specifies the contour of a biological structure reflected in the image indicated by the captured image information obtained by imaging during the irradiation with the special light. Examples of the biological structure of a specific target include a superficial blood vessel structure, a middle layer blood vessel structure, or a deep blood vessel structure. The analysis by the analysis unit 42c is performed in parallel with displaying of the live image.

The analysis image generation unit 42d generates IEE image information for displaying an IEE image indicating the result of the analysis output from the analysis unit 42c, and outputs the generated IEE image information to the display controller 43 (see Fig. 2) as the captured image information. The IEE image is an image in which the contour of the structure of the subject is enhanced based on the imaging signal obtained by imaging during the irradiation with the special light, such as a blue laser. In this case, the special light, such as the blue laser, constitutes light for image-enhanced endoscopy. For example, the IEE image is an image in which the superficial blood vessel structure is enhanced, an image in which the middle layer blood vessel structure is enhanced, an image in which the deep blood vessel structure is enhanced, and the like.

It should be noted that the image generated by the analysis image generation unit 42d is not limited to the captured image and the processed image of the captured image, and may be an image indicating the numerical value (number or accuracy) or the text (type of tumor) based on the analysis by the analysis unit 42c.

As described with reference to Fig. 5, the endoscope apparatus 100 comprises the analysis unit 42c that performs the analysis based on the captured image information obtained by imaging in a second period in which the special light is emitted in the captured image information. On the other hand, the endoscope apparatus 100 displays the live image based on the captured image information obtained by imaging in a first period in which the normal light is emitted in the captured image information. As a result, it is possible to perform the analysis based on the special light while performing the motion picture display based on the normal light.

### <Screen Displayed on Display 7>

Fig. 6 is a diagram showing an example of a screen displayed on the display 7.

The display controller 43 displays, for example, a screen 70 shown in Fig. 6 on the display 7 based on the captured image information output from the signal processing unit 42. The screen 70 includes a main screen 71 and the sub-screen 72.

The live image based on the live image information output from the live image generation unit 42b of the signal processing unit 42 is displayed on the main screen 71. The IEE image based on the IEE image information output from the analysis image generation unit 42d of the signal processing unit 42 is displayed on the sub-screen 72.

As described with reference to Fig. 6, the endoscope apparatus 100 displays the screen 70 including the live image based on the captured image information obtained by imaging in the first period in which the normal light is emitted, and the result of the analysis based on the captured image information obtained by imaging in the second period in which the special light is emitted.

It should be noted that, although the configuration has been described in which a result of the analysis of the analysis unit 42c shown in Fig. 5 is displayed on the display 7 together with the live image, but a usage form of the result of the analysis of the analysis unit 42c is not limited to this. For example, the result of the analysis of the analysis unit 42c may be displayed by a device different from the display 7, or may be transmitted to and stored in the storage unit of the endoscope apparatus 100 or another device.

### <Switching of Illumination Light in Endoscope Apparatus 100>

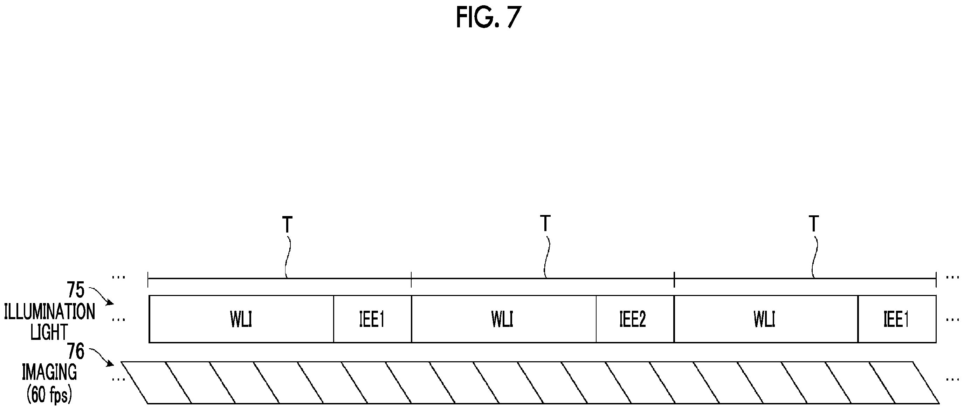

Fig. 7 is a diagram showing an example of switching of the illumination light in the endoscope apparatus 100.

An illumination light timing 75 is a timing at which the light source device 5 emits the illumination light in accordance with the command from the control device 4. The WLI in the illumination light timing 75 is a timing at which the light source device 5 emits the normal light, such as the white light, as the illumination light. The IEE1 in the illumination light timing 75 is a timing at which the light source device 5 emits first special light, such as narrow band light, as the illumination light. The IEE2 in the illumination light timing 75 is a timing at which the light source device 5 emits second special light different from the first special light as the illumination light.

As shown in the illumination light timing 75, the light source device 5 repeatedly executes a predetermined irradiation operation in a period T. The irradiation operation is an operation of emitting the normal light and then emitting the special light (first special light or second special light). In the example shown in Fig. 7, the light source device 5 alternately switches the special light to be emitted between the first special light and the second special light for each period T. It should be noted that the light source device 5 may use only the first special light as the special light for each period T.

An imaging timing 76 is a timing at which the imaging element 23 performs imaging (exposure) in accordance with the command from the control device 4. A vertical direction at the imaging timing 76 indicates a position of the pixel row 62 in the column direction Y (see Fig. 4). As described above, since the imaging element 23 in the present embodiment performs imaging of the rolling shutter system, the imaging timing 76 deviates for each pixel row 62. In the example shown in Fig. 7, the imaging element 23 performs imaging at a frame rate of 60 frames per second (fps).

As shown in the illumination light timing 75 and the imaging timing 76, the first period in which the light source device 5 continuously emits the normal light extends over a plurality of consecutive frames in imaging by the imaging element 23. In addition, the second period in which the light source device 5 continuously emits the special light extends over at least one frame in imaging by the imaging element 23. In the example shown in Fig. 7, the second period extends over the plurality of consecutive frames by the imaging element 23.

As described above, the light source device 5 repeats the operation of continuously emitting the normal light (illumination light having a first characteristic) over the plurality of consecutive imaging operation frames, and then emitting the special light (illumination light having a second characteristic different from the first characteristic). Moreover, as described above, the control device 4 displays the live image (motion picture) on the display 7 based on the captured image (first captured image) obtained during the irradiation with the normal light, and performs the analysis based on the captured image (second captured image) obtained during the irradiation with the special light.

Here, during the irradiation with the special light, the captured image of the normal light cannot be obtained, so that the live image is interrupted, for example. In addition, it is also conceivable to continue the live image by using the captured image of the special light for the imaging frame during the irradiation with the special light, but the live image is temporarily changed from an image of the normal light to an image of the special light, so that there is a sense of discomfort of the display.

On the other hand, the control device 4 reduces the sense of discomfort of the display by displaying the live image of the imaging frame during the irradiation with the special light based on the imaging frame different from the imaging frame. As a result, for example, it is possible to increase the frequency of the irradiation with the special light while suppressing the sense of discomfort of the display, and to improve the accuracy or the real-time property of the analysis using the special light.

It should be noted that the frequency of the irradiation with the special light can be, for example, every 10 frames. It should be noted that the frequency of the irradiation with the special light is not limited to this and can be set optionally. In addition, a period of the irradiation with the special light once may be a period of one frame or may be a period of one frame or more.

### <Change in Display Image due to Switching of Illumination Light>

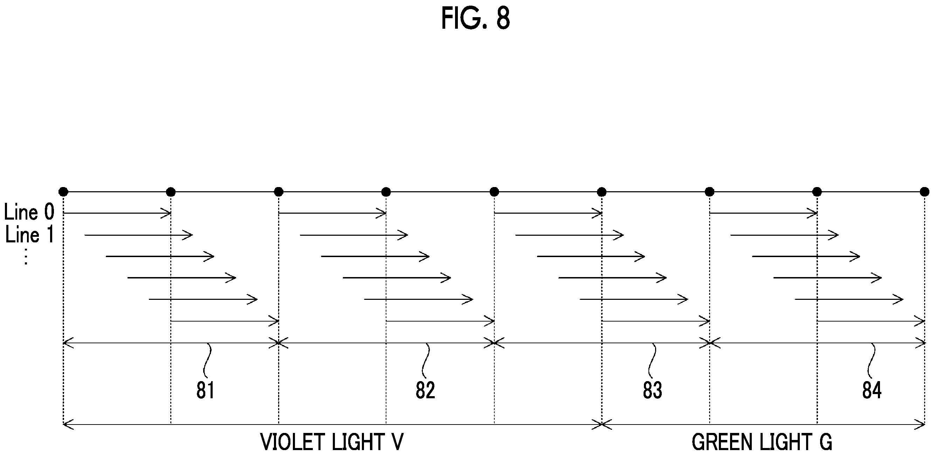

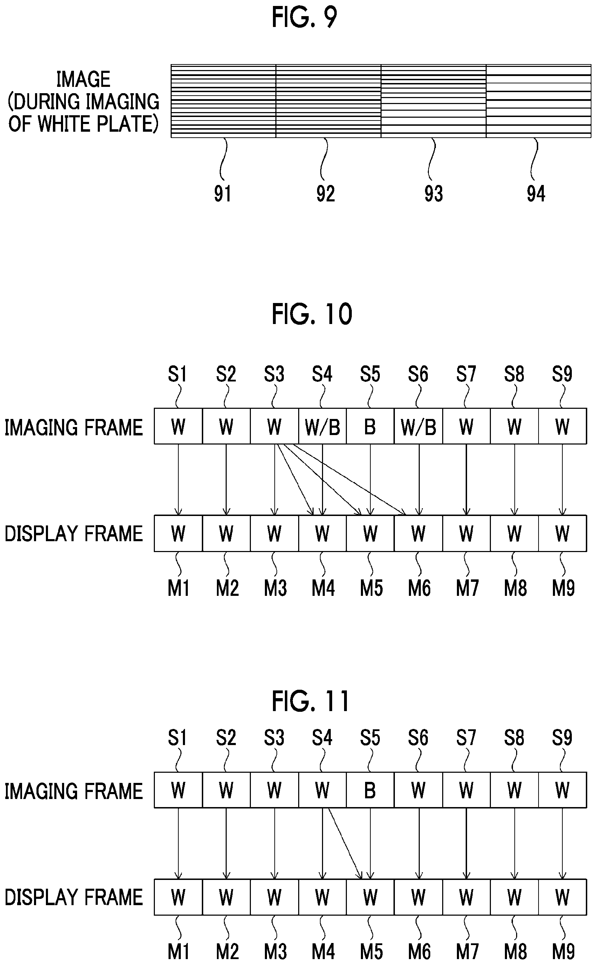

Figs. 8 and 9 are diagrams showing an example of the change in the image due to switching of the illumination light.

Here, as a virtual example, a case will be described in which a white plate is imaged by the imaging element 23 and the illumination light of the light source device 5 is switched from the violet light V to the green light G.

In Fig. 8, a horizontal axis indicates a time, and a vertical axis (Line 0, Line 1, ...) indicates a position in the column direction Y (see Fig. 4) of the pixel row 62 of the imaging element 23 for which the exposure by the rolling shutter is performed.

As described above, the imaging element 23 is the CMOS using the rolling shutter. An arrow to a right direction in Fig. 8 indicates the exposure for each pixel row 62 performed in the imaging element 23. Each of exposure periods 81 to 84 indicated by the arrows in both directions is an exposure period for one field of display on the display 7.

In the example shown in Fig. 8, the illumination light of the light source device 5 is the violet light V in a period from the exposure period 81 to the middle of the exposure period 83. Moreover, from the middle of the exposure period 83, the illumination light of the light source device 5 is switched to the green light G.

Images 91 to 94 shown in Fig. 9 are images generated based on the imaging signals obtained by the exposure in the exposure periods 81 to 84, respectively. In the images 91 to 94, a color is represented by a density of the horizontal lines. Specifically, dense horizontal lines, such as the images 91 and 92, represent violet, and non-dense horizontal lines, such as the image 94, represent green. In the image 93, the density of horizontal lines is decreased from top to bottom, which represents that the color of image 93 is changed from violet to green from top to bottom.

As shown in Figs. 8 and 9, the illumination light of the light source device 5 is switched to the green light G in the middle of the exposure period 83, so that the image 93 corresponding to the exposure period 83 is an image in which violet and green are mixed. In addition, the images 91 and 92 corresponding to the exposure periods 81 and 82 are violet images, and the image 94 corresponding to the exposure period 84 is a green image.

### <Interpolation of Display Frame during Irradiation with Special Light>

Fig. 10 is a diagram showing an example of the interpolation of the display frame during the irradiation with the special light.

In Fig. 10, imaging frames S1 to S9 are consecutive imaging frames obtained by the imaging element 23. In the imaging frames S1 to S9, "W" indicates an imaging frame obtained during the irradiation with the normal light, such as the white light, "B" indicates an imaging frame obtained during the irradiation with the special light, such as a blue laser, and "W/B" indicates an imaging frame obtained during switching between the normal light and the special light, similar to the image 93 shown in Fig. 9.

In the example shown in Fig. 10, each period of the imaging frames S1 to S3 and the imaging frames S7 to S9 is an example of the first period over the plurality of consecutive imaging operation frames. The period of the imaging frame S5 is an example of the second period over at least one imaging operation frame. Each period of the imaging frames S4 and S6 is an example of a third period in which the normal light and the special light are switched.

Display frames M1 to M9 are consecutive display frames displayed by the display 7, and correspond to the imaging frames S1 to S9, respectively. In the display frames M1 to M9, "W" indicates the image of the normal light, such as the white light.

For example, the control device 4 generates the display frame M1 (W) based on the imaging frame S1 (W) obtained during the irradiation with the normal light. Similarly, the control device 4 generates the display frames M2, M3, and M7 to M9 (W), respectively, based on the imaging frames S2, S3, and S7 to S9 (W) obtained during the irradiation with the normal light.

In addition, for the imaging frame S5 (B) obtained during the irradiation with the special light, the control device 4 generates the display frame M5 (W) of the normal light based on the imaging frame S5 and the imaging frame S3 obtained during the irradiation with the normal light and immediately before the imaging frame S5.

The generation of the display frame M5 (W) based on the imaging frames S3 and S5 will be described. Here, a color space of each imaging frame is expressed by using a brightness signal Y, such as YCbCr, and two color difference signals.

For example, based on brightness signals Y3 and Y5 of the imaging frames S3 and S5, the control device 4 calculates a movement vector (motion vector) indicating the movement of a brightness signal YY5 of the imaging frame S5 with reference to the brightness signal Y3 of the imaging frame S3.

Moreover, the control device 4 corrects the imaging frame S3 (W) based on the calculated movement vector to generate the display frame M5 (W) of the normal light. Specifically, the control device 4 generates the display frame M5 (W) of the normal light by applying a movement amount indicated by the movement vector to the color difference signal of the imaging frame S3.

As described above, for a period of the imaging frame S5, the control device 4 generates the display frame M5 based on the movement vector based on brightness information of each of the imaging frame S3 and the imaging frame S5 and the color difference of the imaging frame S3.

In this case, the display frame M5 is the image of the normal light in which the brightness information, such as the contour, corresponds to the imaging frame S5 (B) and the color corresponds to the imaging frame S3 (W). As a result, it is possible to obtain the image of the normal light with less sense of discomfort, which corresponds to the imaging frame S5 (B) obtained during the irradiation with the special light. It should be noted that a method of generating the display frame M5 based on the imaging frames S3 and S5 is not limited to this method, and various methods can be used. For example, the control device 4 may generate, as the display frame M5, an image having the brightness information of the imaging frame S5 and the color difference information of the imaging frame S3.

Similarly, for the imaging frame S4 (W/B) obtained during switching between the normal light and the special light, the control device 4 generates the display frame M4 (W) of the normal light based on the imaging frame S4, and the imaging frame S3 obtained during the irradiation with the normal light and immediately before the imaging frame S4. The generation of the display frame M4 based on the imaging frames S3 and S4 can be performed in the same manner as the generation of the display frame M5 based on the imaging frames S3 and S5.

In addition, for the imaging frame S6 (W/B) obtained during switching between the normal light and the special light, the control device 4 generates the display frame M6 (W) of the normal light based on the imaging frame S6, and the imaging frame S3 obtained during the irradiation with the normal light and immediately before the imaging frame S6. The generation of the display frame M6 based on the imaging frames S3 and S6 can be performed in the same manner as the generation of the display frame M5 based on the imaging frames S3 and S5.

As described above, with the endoscope apparatus 100, even during the irradiation with the special light or during switching between the normal light and the special light, the display frame of the normal light with less sense of discomfort can be generated and displaying the live image of the normal light can be continued.

It should be noted that, for example, regarding the generation of the display frame M5 corresponding to the imaging frame S5, the case has been described in which the display frame M5 of the normal light is generated based on the imaging frame S3 during the irradiation with the normal light and immediately before the imaging frame S5, but the imaging frame during the irradiation with the normal light, which is to used, is not limited to the imaging frame immediately before the imaging frame S5.

For example, the control device 4 may generate the display frame M5 of the normal light based on at least any of the imaging frames S1 to S3 during the irradiation with the normal light and before the imaging frame S5. In addition, the control device 4 may generate the display frame M5 of the normal light based on at least any of the imaging frames S7 to S9 during the irradiation with the normal light and after the imaging frame S5. In addition, the control device 4 may generate the display frame M5 of the normal light based on at least any of the imaging frames S1 to S3 and at least one of the imaging frames S7 to S9.

In addition, the control device 4 may generate the display frame M5 based on the imaging frame having a high correlation with the imaging frame S5 among the imaging frames before and after the imaging frame S5.

The correlation is, for example, a correlation of the brightness signal.

Similarly, for the generation of the display frame M4 corresponding to the imaging frame S4 or the generation of the display frame M6 corresponding to the imaging frame S6, the imaging frame during the irradiation with the normal light, which is used, is not limited to the imaging frame immediately before the target imaging frame.

In addition, although the configuration has been described in which the brightness signal of the imaging frame S5 is used for generating the display frame M5, the configuration is not limited to such a configuration. For example, the control device 4 may use the display frame M3 based on the imaging frame S3 as the display frame M5, or may generate the display frame M5 based on the imaging frames S3 and S7. For example, the control device 4 may use the brightness signal obtained by averaging the brightness signals indicated by the imaging frames S3 and S7 instead of the brightness signal of the imaging frame S5.

Fig. 11 is a diagram showing another example of the interpolation of the display frame during the irradiation with the special light.

Although the configuration has been described in which the rolling shutter is used for the imaging element 23, a configuration may be adopted in which the global shutter is used for the imaging element 23. In this case, by switching between the normal light and the special light in synchronization with the imaging frame, it is possible to prevent the generation of the imaging frame in which the normal light and the special light are mixed.

For example, in the example shown in Fig. 10, among the imaging frames S1 to S9, the irradiation with the special light is performed only in the period of the imaging frame S5, and the irradiation with the normal light is performed in the periods of the other imaging frames. In this case, the display frame, which is an interpolation target, can be only the display frame M5 corresponding to the imaging frame S5 among the display frames M1 to M9.

Specifically, the control device 4 generates the display frames imaging frames M1 to M4 and M6 to M9 (W) based on the imaging frames S1 to S4 and S6 to S9 (W) obtained during the irradiation with the normal light, respectively. In addition, for the imaging frame S5 (B) obtained during the irradiation with the special light, the control device 4 generates the display frame M5 (W) of the normal light based on the imaging frame S5 and the imaging frame S3 obtained during the irradiation with the normal light and immediately before the imaging frame S5.

As a result, similar to the example shown in Fig. 10, even during the irradiation with the special light or during switching between the normal light and the special light, the display frame of the normal light with less sense of discomfort can be generated and displaying the live image of the normal light can be continued.

It should be noted that, even in the configuration in which the global shutter is used for the imaging element 23, the imaging frame in which the normal light and the special light are mixed may be generated. In such a case, the imaging frame in which the normal light and the special light are mixed may be a target for the interpolation.

### (Another Example of Analysis)

Although the extraction of the contour of the captured image has been described as the analysis by the analysis unit 42c (signal processing unit 42) based on the captured image information obtained by imaging during the irradiation with the special light, the analysis by the analysis unit 42c is not limited to this.

For example, the analysis unit 42c may analyze an insertion shape of the endoscope 1 as the analysis described above. Specifically, the analysis of the insertion shape of the endoscope 1 is specifying of the insertion shape of the insertion part 10 of the endoscope 1 inserted into the subject. For example, the analysis unit 42c specifies the insertion shape of the endoscope 1 based on the change in the captured image information obtained by imaging during the irradiation with the special light. The analysis image generation unit 42d generates image information for displaying an image indicating the insertion shape of the endoscope 1 specified by the analysis unit 42c. As a result, the image indicating the insertion shape of the endoscope 1 is displayed on the sub-screen 72, so that the operator of the endoscope 1 can easily insert the insertion part 10 of the endoscope 1 into the subject.

Alternatively, the analysis unit 42c may detect a region-of-interest inside the subject into which the endoscope 1 is inserted as the analysis described above. For example, the analysis unit 42c detects the region-of-interest inside the subject from the image indicated by the captured image information obtained by imaging during the irradiation with the special light. The region-of-interest is a region that is recommended for attention in the observation of the inside of the subject, such as a region that is likely to be a lesion. The analysis image generation unit 42d generates image information for displaying a region-of-interest-enhanced image in which the region-of-interest detected by the analysis unit 42c is enhanced in the image indicated by the captured image information obtained by imaging during the irradiation with the special light. As a result, the region-of-interest-enhanced image is displayed on a sub-screen 72, and the operator of the endoscope 1 can easily recognize the region-of-interest inside the subject. Alternatively, the analysis image generation unit 42d may generate image information for displaying color difference-expanded image subjected to color difference expansion processing of expanding a color difference between an abnormal site (lesion site), which is the region-of-interest, and a normal site in the image indicated by the captured image information obtained by imaging during the irradiation with the special light. As a result, the color difference-expanded image is displayed on the sub-screen 72, and the operator of the endoscope 1 can easily distinguish between the abnormal site and the normal site inside the subject.

Alternatively, the analysis unit 42c may select a similar case image as the analysis described above. For example, the analysis unit 42c selects a case image similar to the captured image information obtained by imaging during the irradiation with the special light by searching a database accessible to the endoscope apparatus 100. The analysis image generation unit 42d generates image information for displaying an image indicating a selection result by the analysis unit 42c. The selection result by the analysis unit 42c may be the case image itself selected by the analysis unit 42c, or may be information, such as diagnosis result, relating to the case image associated with the case image selected by the analysis unit 42c in the database. As a result, the selection result of the similar case image is displayed on the sub-screen 72, and the operator of the endoscope 1 can easily compare a state inside the subject under observation with the similar case.

Alternatively, the analysis unit 42c may perform determination of a tumor and a non-tumor as the analysis described above. For example, the analysis unit 42c determines whether or not a biological region reflected in the image indicated by the captured image information obtained by imaging during the irradiation with the special light is the tumor. The analysis image generation unit 42d generates image information for displaying an image indicating a determination result by the analysis unit 42c. The determination result by the analysis unit 42c may be information indicating whether or not the biological region reflected in the most recently captured image is the tumor, or may be information indicating the number of the biological regions determined to be the tumor from the start of the current examination. As a result, the determination result of the tumor and the non-tumor is displayed on the sub-screen 72, and it is possible to support the observation or the operation of the endoscope 1 by the operator of the endoscope 1.

Alternatively, the analysis unit 42c may specify a state of an organ as the analysis described above. For example, the analysis unit 42c specifies the state of the organ reflected in the image indicated by the captured image information obtained by imaging during the irradiation with the special light. Examples of the state of the organ include the oxygen saturation for each region, the thickness, the density, pattern, and the uniformity of the blood vessel structure, the surface structure of the large intestine (for example, pit pattern structure), or the surface structure of the duodenum (for example, villous structure). The analysis image generation unit 42d generates image information for displaying an image indicating a specifying result by the analysis unit 42c. For example, the analysis image generation unit 42d generates an oxygen saturation image obtained by imaging the oxygen saturation for each specified region. As a result, the specifying result of the state of the organ is displayed on the sub-screen 72, and it is possible to support the observation or the operation of the endoscope 1 by the operator of the endoscope 1.

Alternatively, the analysis unit 42c may generate a planned separation line as the analysis described above. For example, the analysis unit 42c decides the planned separation line (demarcation line) which is the line to be separated to remove the tumor in the biological region reflected in the image indicated by the captured image information obtained by imaging during the irradiation with the special light. The analysis image generation unit 42d generates image information for displaying an image to which the planned separation line decided by the analysis unit 42c is attached in the image indicated by the captured image information obtained by imaging during the irradiation with the special light. As a result, the image to which the planned separation line is attached is displayed on the sub-screen 72, and the operator of the endoscope 1 can easily recognize the planned separation line inside the subject.

### (Modification Examples of First Period, Second Period, and Period T)

Although the configuration has been described in which the length of each of the first period in which the normal light is emitted and the second period in which the special light is emitted is fixed in the repetition for each period T, but the length of each of the first period in which the normal light is emitted and the second period in which the special light is emitted may not be fixed (may be variable) in the repetition for each period T. For example, a ratio of the lengths of the first period and the second period in one period T may be 3:1, and a ratio of the lengths of the first period and the second period in the other period T may be 3:2.

In addition, although the case has been described in which the period T, which is the repetition period of the operation of emitting the normal light and the special light, is fixed, the period T may be variable. In addition, the configuration has been described in which the normal light is first emitted and then the special light is emitted in the period T, a configuration may be adopted in which the special light is first emitted and then the normal light is emitted in the period T.

In addition, the spectrum of the normal light may be fixed in the repetition for each period T or may be variable in the repetition for each period T. Similarly, the spectrum of the special light may be fixed in the repetition for each period T or may be variable in the repetition for each period T.

In addition, although the configuration has been described in which the second period in which the special light is emitted is immediately after the first period in which the normal light is emitted, a non-irradiation period in which the light source device 5 does not emit the illumination light may be present between the first period and the second period.

In addition, a configuration may be adopted in which narrow-band short-wavelength dimming light and white light are simultaneously emitted as the normal light or the special light described above. As a result, minute differences in color are enhanced and displayed, and the observation, such as inflammation observation or pick-up observation, is facilitated.

### (Another Embodiment of Endoscope System)

The endoscope apparatus 100 has been described as an example of the endoscope system according to the embodiment of the present invention, the endoscope system according to the embodiment of the present invention may be realized by a plurality of devices connected to each other via the network. For example, a configuration may be adopted in which at least a part of the processing by the control device 4 described above is executed by another device connected to the endoscope apparatus 100 via the network.

### (Control Program)

A control program, which is stored in the ROM of the control device 4, is stored in a program computer-readable non-transitory storage medium. Examples of such a "computer-readable storage medium" include an optical medium, such as a compact disc-ROM (CD-ROM), and a magnetic storage medium, such as a universal serial bus (USB) memory, or a memory card. In addition, such a program can be provided by being downloaded via a network.

As described above, the following matters are disclosed in the present specification.
(1)
   An endoscope system comprising a light source, an imaging element, a display, and a processor, in which the light source is able to switch between a plurality of types of illumination light having different characteristics to perform irradiation, and the processor repeats an operation of continuously emitting illumination light having a first characteristic from the light source in a first period over a plurality of consecutive imaging operation frames, and then emitting illumination light having a second characteristic different from the first characteristic from the light source in a second period over at least one imaging operation frame, and acquires a captured image from the imaging element, and causes the display to display, for the first period, the motion picture based on the captured image of the first period and to display, for the second period, the motion picture based on the captured image of a period different from the second period.
(2)
   The endoscope system according to (1), in which the processor displays, for a third period in which the illumination light having the first characteristic and the illumination light having the second characteristic are switched, the motion picture based on the captured image of a period different from the third period.
(3)
   The endoscope system according to (1), in which the processor displays, for the third period, the motion picture based on the captured image of a period different from the third period and the second period.
(4)
   The endoscope system according to any one of (1) to (3), in which the processor displays, for the second period, the motion picture based on a first captured image of the second period and a second captured image of the first period different from the second period.
(5)
   The endoscope system according to (4), in which the processor displays, for the second period, the motion picture based on a movement vector based on each piece of brightness information of the first captured image and the second captured image, and a color difference of the second captured image.
(5)
   The endoscope system according to (4), in which the processor displays, for the second period, the motion picture based on a movement vector based on each piece of brightness information of a second captured image, and a color difference of at least one second captured image.
(6)
   The endoscope system according to any one of (1) to (5), in which the captured image obtained from the imaging element in the second period is subjected to image analysis.
(7)
   The endoscope system according to (6), in which the image analysis is performed in parallel with displaying the motion picture.
(8)
   The endoscope system according to (6) or (7), in which the processor displays a screen including the motion picture and a result of the image analysis on the display.
(9)
   The endoscope system according to any one of (6) to (8), in which the image analysis includes analysis of an insertion shape of an endoscope including the imaging element.
(10)
   The endoscope system according to any one of (6) to (9), in which the image analysis includes extraction of a contour of the captured image.
(11)
   The endoscope system according to any one of (6) to (10), in which the image analysis includes detection of a region-of-interest inside a subject into which an endoscope including the imaging element is inserted.
(12)
   The endoscope system according to any one of (6) to (11), in which the image analysis includes selection of a similar case image.
(13)
   The endoscope system according to any one of (6) to (12), in which the image analysis includes determination of a tumor and a non-tumor.
(14)
   The endoscope system according to any one of (6) to (13), in which the image analysis includes specifying a state of an organ.
(15)
   The endoscope system according to any one of (6) to (14), in which the image analysis includes generation of a planned separation line.
(16)
   The endoscope system according to any one of (1) to (15), in which a length of each of the first period and the second period is fixed in repetition of the operation or is variable in repetition of the operation.
(17)
   The endoscope system according to any one of (1) to (16), in which spectra of the illumination light having the first characteristic and the illumination light having the second characteristic are fixed in repetition of the operation or is variable in repetition of the operation.
(18)
   The endoscope system according to any one of (1) to (17), in which a non-irradiation period of the light source is present between the first period and the second period.
(19)
   The endoscope system according to any one of (1) to (18), in which the imaging element performs an imaging operation of a rolling shutter system.
(20)
   The endoscope system according to any one of (1) to (18), in which the imaging element performs an imaging operation of a global shutter system.
(21)
   The endoscope system according to any one of (1) to (20), in which the first period is a period longer than the second period.
(22)
   The endoscope system according to any one of (1) to (21), in which the illumination light having the first characteristic and the illumination light having the second characteristic are white light or light for image-enhanced endoscopy.
(23)
   A control method of an endoscope system including a light source, an imaging element, a display, and a processor, in which the light source is able to switch between a plurality of types of illumination light having different characteristics to perform irradiation, the method comprising repeating an operation of causing the light source to continuously emit illumination light having a first characteristic in a first period over a plurality of consecutive imaging operation frames, and then emit illumination light having a second characteristic different from the first characteristic in a second period over at least one imaging operation frame, and acquiring a captured image from the imaging element, and causing the display to display, for the first period, the motion picture based on the captured image of the first period and to display, for the second period, the motion picture based on the captured image of a period different from the second period.
(24)
   A control program that controls an endoscope system including a light source, an imaging element, a display, and a processor, in which the light source is able to switch between a plurality of types of illumination light having different characteristics to perform irradiation, the program causing a computer to execute a process comprising repeating an operation of causing the light source to continuously emit illumination light having a first characteristic in a first period over a plurality of consecutive imaging operation frames, and then emit illumination light having a second characteristic different from the first characteristic in a second period over at least one imaging operation frame, and acquiring a captured image from the imaging element, and causing the display to display, for the first period, the motion picture based on the captured image of the first period and to display, for the second period, the motion picture based on the captured image of a period different from the second period.

According to the present invention, it is possible to provide the endoscope system, the control method, and the control program that can perform imaging with the special light while suppressing the deterioration of the quality of the motion picture display.

### Explanation of References

1: endoscope
4: control device
5: light source device
6: input unit
7: display
10: insertion part
10A: flexible part
10B: bendable part
10C: distal end part
11: operating part
12: angle knob
13: universal cord
13A: connector portion
13B: connector portion
21: objective lens
22: lens group
23: imaging element
25: memory
26, 41: communication I/F
27: imaging driving unit
42: signal processing unit
42a: captured image information generation unit
42b: live image generation unit
42c: analysis unit
42d: analysis image generation unit
43: display controller
44: system control unit
45: recording medium
50: illumination lens
51: light source processor
52: light source unit
52a: V-LED
52b: B-LED
52c: G-LED
52d: R-LED
53: light guide
54: optical path coupling unit
60: imaging surface
61: pixel
61: each pixel
62: pixel row
62: each pixel row
63: drive circuit
64: signal processing circuit
70: screen
71: main screen
72: sub-screen
75: illumination light timing
76: imaging timing
81 to 84: exposure period
91 to 94: image
100: endoscope apparatus
S1 to S9: imaging frame
M1 to M9: display frame

## Claims

1. An endoscope system comprising:
a light source;
an imaging element;
a display; and
a processor,
wherein the light source is able to switch between a plurality of types of illumination light having different characteristics to perform irradiation, and
the processor is configured to:
repeat an operation of continuously emitting illumination light having a first characteristic from the light source in a first period over a plurality of consecutive imaging operation frames, and then emitting illumination light having a second characteristic different from the first characteristic from the light source in a second period over at least one imaging operation frame, and
acquire a captured image from the imaging element, and cause the display to display, for the first period, the motion picture based on the captured image of the first period and to display, for the second period, the motion picture based on the captured image of a period different from the second period.

2. The endoscope system according to claim 1,
wherein the processor is configured to display, for a third period in which the illumination light having the first characteristic and the illumination light having the second characteristic are switched, the motion picture based on the captured image of a period different from the third period.

3. The endoscope system according to claim 2,
wherein the processor is configured to display, for the third period, the motion picture based on the captured image of a period different from the third period and the second period.

4. The endoscope system according to any one of claims 1 to 3,
wherein the processor is configured to display, for the second period, the motion picture based on a first captured image of the second period and a second captured image of the first period different from the second period.

5. The endoscope system according to claim 4,
wherein the processor is configured to display, for the second period, the motion picture based on a movement vector based on each piece of brightness information of the first captured image and the second captured image, and a color difference of the second captured image.

6. The endoscope system according to any one of claims 1 to 3,
wherein the processor is configured to display, for the second period, the motion picture based on a movement vector based on each piece of brightness information of a second captured image of the first period different from the second period, and a color difference of at least one second captured image.

7. The endoscope system according to any one of claims 1 to 6,
wherein the captured image obtained from the imaging element in the second period is subjected to image analysis.

8. The endoscope system according to claim 7,
wherein the image analysis is performed in parallel with displaying the motion picture.

9. The endoscope system according to claim 7 or 8,
wherein the processor is configured to display a screen including the motion picture and a result of the image analysis on the display.

10. The endoscope system according to any one of claims 7 to 9,
wherein the image analysis includes analysis of an insertion shape of an endoscope including the imaging element.

11. The endoscope system according to any one of claims 7 to 10,
wherein the image analysis includes extraction of a contour of the captured image.

12. The endoscope system according to any one of claims 7 to 11,
wherein the image analysis includes detection of a region-of-interest inside a subject into which an endoscope including the imaging element is inserted.

13. The endoscope system according to any one of claims 7 to 12,
wherein the image analysis includes selection of a similar case image.

14. The endoscope system according to any one of claims 7 to 13,
wherein the image analysis includes determination of a tumor and a non-tumor.

15. The endoscope system according to any one of claims 7 to 14,
wherein the image analysis includes specifying a state of an organ.

16. The endoscope system according to any one of claims 7 to 15,
wherein the image analysis includes generation of a planned separation line.

17. The endoscope system according to any one of claims 1 to 16,
wherein a length of each of the first period and the second period is fixed in repetition of the operation or is variable in repetition of the operation.

18. The endoscope system according to any one of claims 1 to 17,
wherein spectra of the illumination light having the first characteristic and the illumination light having the second characteristic are fixed in repetition of the operation or is variable in repetition of the operation.

19. The endoscope system according to any one of claims 1 to 18,
wherein a non-irradiation period of the light source is present between the first period and the second period.

20. The endoscope system according to any one of claims 1 to 19,
wherein the imaging element performs an imaging operation of a rolling shutter system.

21. The endoscope system according to any one of claims 1 to 20,
wherein the imaging element performs an imaging operation of a global shutter system.

22. The endoscope system according to any one of claims 1 to 21,
wherein the first period is a period longer than the second period.

23. The endoscope system according to any one of claims 1 to 22,
wherein the illumination light having the first characteristic and the illumination light having the second characteristic are white light or light for image-enhanced endoscopy.

24. A control method of an endoscope system including a light source, an imaging element, a display, and a processor, in which the light source is able to switch between a plurality of types of illumination light having different characteristics to perform irradiation, the method comprising:
repeating an operation of causing the light source to continuously emit illumination light having a first characteristic in a first period over a plurality of consecutive imaging operation frames, and then emit illumination light having a second characteristic different from the first characteristic in a second period over at least one imaging operation frame; and
acquiring a captured image obtained from the imaging element, and causing the display to display, for the first period, the motion picture based on the captured image of the first period and to display, for the second period, the motion picture based on the captured image of a period different from the second period.

25. A control program that controls an endoscope system including a light source, an imaging element, a display, and a processor, in which the light source is able to switch between a plurality of types of illumination light having different characteristics to perform irradiation, the program causing a computer to execute a process comprising:
repeating an operation of causing the light source to continuously emit illumination light having a first characteristic in a first period over a plurality of consecutive imaging operation frames, and then emit illumination light having a second characteristic different from the first characteristic in a second period over at least one imaging operation frame; and
acquiring a captured image obtained from the imaging element, and causing the display to display, for the first period, the motion picture based on the captured image of the first period and to display, for the second period, the motion picture based on the captured image of a period different from the second period.
